# EUROPEAN PATENT APPLICATION

(11) **EP 1 625 835 A1**
(43) Date of publication of application: **15.02.2006**
(21) Application number: 04733118.6
(22) Date of filing: 14.05.2004
(51) Int. Cl.: A61F 2/44

(54) **INTERSPINAL SPACER**

(30) Priority: 16.05.2003 JP 2003139471
(71) Applicant: Pentax Corporation, Tokyo 174-8639 (JP); Iwasaki, Yoshinobu, Sapporo-shi, Hokkaido 005-0801 (JP); Hida, Kazutoshi, Sapporo-shi, Hokkaido 064-0921 (JP)
(72) Inventor: Nakajima, Takehiko, Itabashi-ku, Tokyo 174-8639 (JP); Iwasaki, Yoshinobu, Sapporo-shi, Hokkaido 005-0801 (JP); Hida, Kazutoshi, Sapporo-shi, Hokkaido 064-0921 (JP)
(74) Representative: Schaumburg, Thoenes, Thurn, Landskron
(86) International application number: PCT/JP2004/006884
(87) International publication number: WO 2004/100840

(57) **Abstract**

An object of the present invention is to provide an interspinous spacer which can be reliably held between adjacent spinous processes to maintain an appropriate space between the adjacent spinous processes, and in particular to provide an interspinous spacer which allows a less invasive operation to be carried out without extensive resection of bone or soft tissue. The interspinous spacer 1 is adapted to be used by inserting it into a space between a spinous process 101 and a spinous process 102, and it is formed from a block body having a recess 21 for receiving a part of each of the spinous processes in a state that the block body is inserted into the space between the spinous processes 101 and 102. The recess 21 is formed in the peripheral surface of the block body 2. The recess 21 has an arc-shaped bottom. The depth of the recess 21 is in the range of 0.5 to 10 mm, and the width of the recess is in the range of 1 to 15 mm. Further, the block body 2 is formed into a substantially cylindrical shape.

## Description

### FIELD OF THE INVENTION

The present invention relates to an interspinous spacer.

### BACKGROUND OF THE INVENTION

Spinal canal stenosis is caused by degeneration of an intervertebral disk interposed between adjacent vertebral bodies, degenerative facet joint disease, secondary deformation of a vertebral body, spinal deformation, or the like, and results in cauda equina/nerve root disorders.

One approach for treating such spinal canal stenosis includes interbody fusion in which a degenerated intervertebral disk is removed from between adjacent vertebral bodies, and then an autologous bone graft is implanted into the intervertebral space to fuse the two vertebral bodies together.

However, in a case where only bone grafting into an intervertebral space is carried out, there is a possibility that spinal instability is caused by resorption of a bone graft until bone fusion is achieved.

As a method for preventing such spinal instability, there is known a method in which an interspinous spacer is inserted between adjacent spinous processes.

Such an interspinous spacer needs to support vertebral bodies with stability and have biocompatibility. From these viewpoints, various interspinous spacers have been proposed as a result of investigation of their constituent materials and shapes (see, for example, USP No. 5,645,599).

An interspinous spacer disclosed in this publication comprises a substantially U-shaped body intended to have resiliency and two pairs of brackets for fixing the U-shaped body between adjacent spinous processes.

However, such an interspinous spacer involves a problem in that normal bone tissue is damaged when the brackets are secured to adjacent spinous processes with screws to fix the spacer between the adjacent spinous processes. In addition, there is also a problem in that the interspinous spacer is complex in shape, which complicates surgical procedures.

### SUMMARY OF THE INVENTION

It is therefore an object of the present invention to provide an interspinous spacer which can be reliably held between adjacent spinous processes to maintain an appropriate space between the adjacent spinous processes. Particularly, it is an object of the present invention to provide an interspinous spacer which allows a less invasive operation to be carried out without extensive resection of bone or soft tissue.

In order to achieve the above-mentioned object, the present invention is directed to an interspinous spacer adapted to be used by being inserted into a space between adjacent spinous processes. The interspinous spacer comprises a block body having a recess for receiving a part of each of the spinous processes in a state that the block body is inserted into the space between the adjacent spinous processes.

According to the interspinous spacer of the present invention, since the spacer is held in the space between the adjacent spinous processes reliably, thereby enabling an appropriate space between the adjacent spinous processes to be maintained.

In the present invention, it is preferred that the recess is provided around the entire circumference of the block body. This makes it possible to hold the spacer between the adjacent spinous processes more reliably. Further, there is an advantage in that positioning of the spacer can be carried out easily when the interspinous spacer is inserted between the adjacent spinous processes.

Further, it is also preferred that the recess has an arc-shaped bottom. This makes it possible to more reliably prevent surrounding tissue from being damaged either when or after the spacer is inserted between the adjacent spinous processes.

Further, it is also preferred that the depth of the recess is in the range of 0.5 to 10 mm. By setting the depth of the recess to a value within the above range, it is possible to more reliably hold the spacer between the adjacent spinous processes.

Further, it is also preferred that the width of the recess is in the range of 1 to 15 mm. By setting the width of the recess to a value within the above range, it is possible to more reliably hold the spacer between the adjacent spinous processes.

Further, it is also preferred that the block body is formed into a substantially cylindrical shape. This makes it possible to prevent surrounding tissue from being easily damaged by the spacer either when or after the spacer is inserted between the adjacent spinous processes.

Further, it is also preferred that the block body is formed with a hollow portion used for passing a fixing member when the interspinous spacer is fixed between the adjacent spinous processes. By fixing the interspinous spacer in such a manner, it is possible to more reliably hold the spacer between the adjacent spinous processes.

Furthermore, it is also preferred that the block body has rounded-corners. This makes it possible to prevent surrounding tissue from being easily damaged by the spacer either when or after the spacer is inserted between the adjacent spinous processes.

Further, it is also preferred that the porosity of the block body is 50% or less. This makes it possible for the block body to have an appropriate strength.

Further, it is also preferred that the block body is formed of a material having biocompatibility. This makes it possible to use the interspinous spacer of the present invention to a human body suitably.

In this case, it is preferred that the block body is mainly formed of a ceramic material. This makes it possible to provide a block body having excellent workability.

Further, in this case, it is also preferred that the ceramic material is a calcium phosphate-based compound. This makes it possible for the block body to have excellent biocompatibility.

Furthermore, in this case, it is also preferred that the calcium phosphate-based compound has a Ca/P ratio of 1.0 to 2.0. This also makes it possible for the block body to have excellent biocompatibility.

Moreover, in this case, it is also preferred that the calcium phosphate-based compound is hydroxyapatite. This also makes it possible for the block body to have especially excellent biocompatibility.

These and other objects, structures and results of the present invention will be apparent more clearly when the following detailed description of the preferred embodiment is considered taken in conjunction with the appended drawings.

### BRIEF DESCRIPTION OF THE DRAWINGS

Fig. 1 is a perspective view which shows an embodiment of a block body constituting the interspinous spacer according to the present invention.
Fig. 2(a) is a plan view of the block body shown in Fig. 1 and Fig. 2(b) is a side view of the block body shown in Fig. 1.
Fig. 3 is an illustration showing a state in which the interspinous spacer according to the present invention is used.
Fig. 4 is an illustration showing a state in which the interspinous spacer according to the present invention is used.
Fig. 5(a) to Fig. 5(c) are illustrations which explain one example of surgical procedures for inserting the interspinous spacer according to the present invention between adjacent spinous processes.

### BEST MODE FOR CARRYING OUT THE INVENTION

Hereinbelow, an interspinous spacer according to the present invention will be described in detail with reference to a preferred embodiment shown in the accompanying drawings.

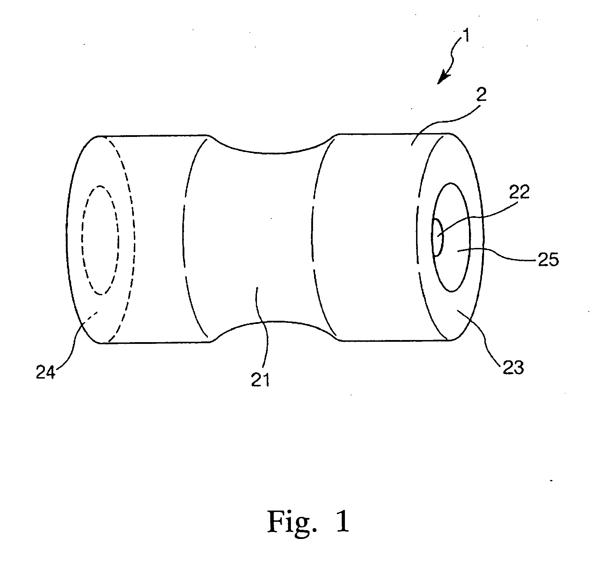

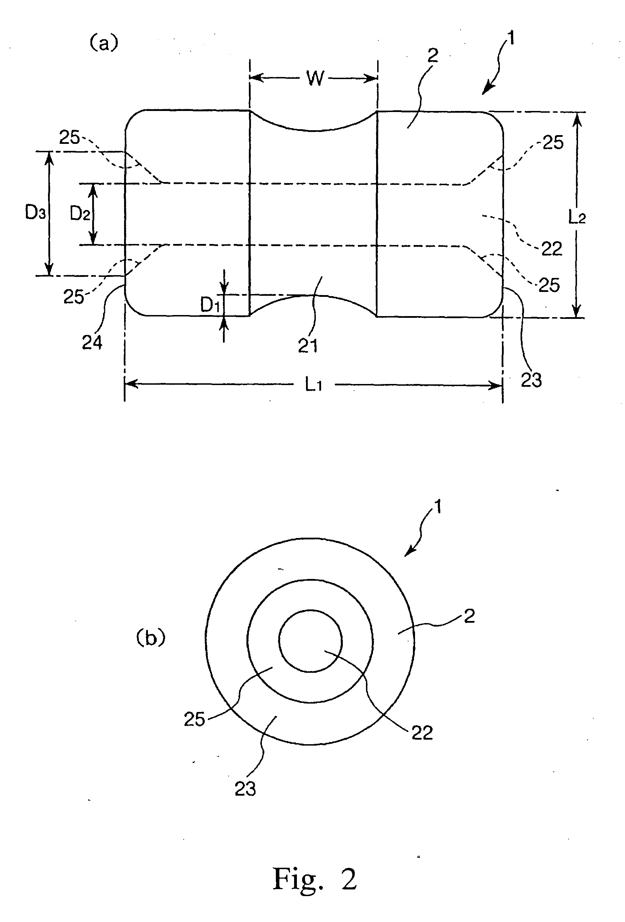

Fig. 1 is a perspective view which shows an embodiment of a block body constituting the interspinous spacer according to the present invention, Fig. 2(a) is a plan view of the block body shown in Fig. 1, Fig. 2(b) is a side view of the block body shown in Fig. 1, and Figs. 3 and 4 are illustrations each showing a state in which the interspinous spacer according to the present invention is used.

In the following description, all directional references are based on a state in which the interspinous spacer is placed between adjacent spinous processes of a patient, unless otherwise noted.

Specifically, the cranial side of a patient (i.e., the upper side in Fig. 1, Figs. 2(a) and 2(b), Fig. 3, and Fig. 4) will be referred to as the "upper side", and the caudal side of a patient (i.e., the lower side in Fig. 1, Figs. 2(a) and 2(b), Fig. 3, and Fig. 4) will be referred to as the "lower side".

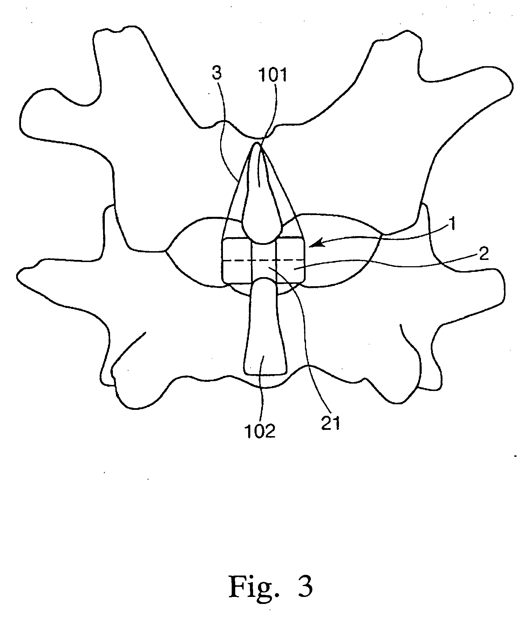

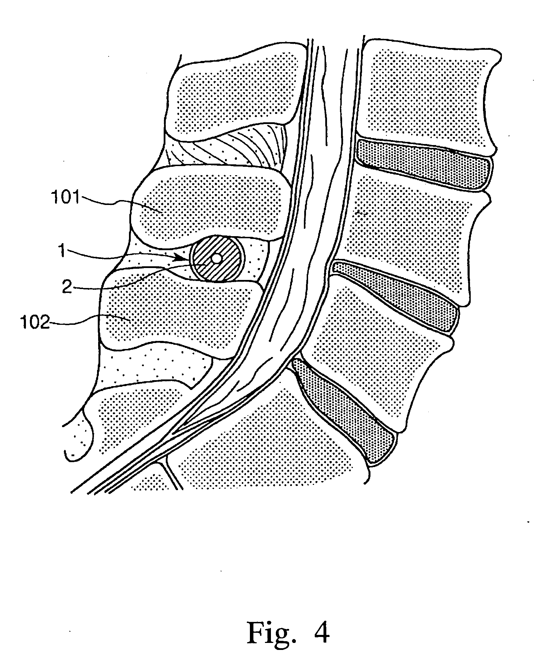

As shown in Figs. 3 and 4, an interspinous spacer 1 according to the present invention is adapted to be inserted in a space between a spinous process 101 and a spinous process 102 (hereinafeter, also referred to as "interspinous space"). In a state where the interspinous spacer 1 is placed in an interspinous space (hereinafter, also referred to as "state of insertion"), an appropriate space (that is, an appropriate distance) between the spinous process 101 and the spinous process 102 is maintained.

As shown in Figs. 1 and 2, the interspinous spacer 1 (hereinafter, also simply referred to as "spacer 1") is composed of a block body 2 having a block-like shape.

As shown in the drawings, the block body 2 has a substantially cylindrical shape, and includes a recess 21 and a hollow portion 22. The block body 2 is formed into an integral body.

As shown in Figs. 3 and 4, the recess 21 has the function of receiving a part of each of the upper and lower spinous processes (that is, each of the adjacent spinous processes) in a state where the spacer 1 is placed in an interspinous space.

As described above, the present invention has a feature in that the block body 2 constituting the spacer 1 includes the recess 21 for receiving a part of each of the upper and lower spinous processes in a state that the spacer 1 is placed between the adjacent spinous processes. According to such a structure, the spacer 1 is held in an interspinous space reliably, thereby enabling an appropriate space between adjacent spinous processes to be maintained. Particularly, by using a spacer having such a structure for treatment, it is possible to carry out a less invasive operation without extensive resection of bone or soft tissue.

According to the present embodiment shown in the drawings, the recess 21 is provided in the peripheral surface of the substantially cylindrical-shaped block body 2 in substantially the middle thereof in an axial direction. This makes it possible to reliably hold the spacer 1 in an interspinous space.

Further, according to the present embodiment, the recess 21 has an arc-shaped bottom. This makes it possible to more reliably prevent surrounding tissue from being damaged by the spacer either when or after the spacer 1 is inserted into an interspinous space.

The recess 21 is not particularly limited as long as it has the function of receiving a part of each of the upper and lower spinous processes in a state that the spacer 1 is placed between the adjacent spinous processes. The recess 21 may be partially provided in the block body 2 constituting the spacer 1. However, as shown in the drawings, it is preferred that the recess 21 be provided around the entire circumference of the block body 2 constituting the spacer 1. This makes it possible to more reliably hold the spacer 1 between adjacent spinous processes. In addition, such a structure is advantageous in that positioning of the spacer 1 is easily carried out when the spacer 1 is inserted into an interspinous space. Further, the recess 21 having such a structure can be easily formed when the spacer 1 is manufactured.

The depth of the recess 21 represented by "D₁" in Fig. 2 (a) is preferably in the range of 0.5 to 10 mm, more preferably in the range of 1 to 2 mm. By setting the depth of the recess 21 to a value within the above range, it is possible to more reliably hold the spacer 1 between adjacent spinous processes. If the depth of the recess 21 is less than the above lower limit value, there is a possibility that the spacer 1 is not securely held between adjacent spinous processes. On the other hand, if the depth of the recess 21 exceeds the above upper limit value, there is a possibility that the strength of the spacer 1 itself is decreased or an appropriate space between adjacent spinous processes is not maintained.

The width of such a groove-like recess 21 represented by "W" in Fig. 2(a) is preferably in the range of 1 to 15 mm, more preferably in the range of 5 to 10 mm. By setting the width of the recess 21 to a value within the above range, it is possible to more reliably hold the spacer 1 in an interspinous space. If the width of the recess 21 is less than the above lower limit value, there is a case that it is difficult for the recess 21 to properly receive spinous processes. On the other hand, if the width of the recess 21 exceeds the above upper limit value, there is a possibility that the spacer 1 is not securely held between adjacent spinous processes.

As described above, the interspinous spacer 1 (that is, the block body 2) according to the present embodiment has a substantially cylindrical shape, and therefore surrounding tissue is not easily damaged by the spacer 1 either when or after the spacer 1 is inserted in an interspinous space.

The spacer 1 is adapted to be fixed between adjacent spinous processes, but the spacer 1 may be fixed in such a manner that it has a certain degree of freedom of movement. By allowing the spacer 1 to have a certain degree of freedom of movement, the spacer 1 can be moved in response to the movements (postures) of a human body, thereby reducing burdens on the human body.

As shown in Figs. 1 and 2, the hollow portion 22 is provided so as to pass through the block body 2 from near the middle of a side surface 23 to near the middle of a side surface 24. That is, the hollow portion 22 is located in substantially the middle of the block body 2 so as to be in parallel with the longitudinal direction of the block body 2 (that is, in parallel with the axial direction of the cylinder).

The hollow portion 22 can be used for, for example, passing a fixing member 3 through the block body 2 when the interspinous spacer 1 is fixed between adjacent spinous processes with the fixing member 3. By fixing the interspinous spacer 1 in such a manner, it is possible to more reliably hold the spacer 1 in an interspinous space. In addition, the hollow portion 22 can also be used for fixing the interspinous spacer 1 to an insertion tool for inserting the spacer 1 into an interspinous space.

As shown in Fig. 2, the hollow portion 22 has an end portion, which slopes toward an opening, on either side thereof. Specifically, the hollow portion 22 has a gradually increasing portion 25 on either side thereof, and the gradually increasing portion 25 slopes toward an opening (that is, toward the end of the block body 2) so as to increase the cross sectional area of the hollow portion 22 in a direction perpendicular to the axial direction of the block body 2 having a substantially cylindrical shape. By providing such a gradually increasing portion 25, it is possible to, for example, prevent the fixing member 3 from coming loose or effectively prevent the fixing member 3 from being damaged due to the movement of the spacer 1 when the fixing member 3 is passed through the hollow portion 22 to fix the spacer 1 between adjacent spinous processes with the fixing member 3. In addition, the gradually increasing portion 25 functions as a guide portion when the fixing member 3 is passed through the hollow portion 22.

The diameter of the hollow portion 22 represented by "D₂" in Fig. 2(a) is not particularly limited, but is preferably in the range of 1 to 5 mm, more preferably in the range of 2 to 4 mm. If the diameter of the hollow portion 22 is less than the above lower limit value, there is a case that it is difficult to pass the fixing member 3 through the hollow portion 22. On the other hand, if the diameter of the hollow portion 22 exceeds the above upper limit value, there is a possibility that the interspinous spacer 1 cannot have adequate strength required for an interspinous spacer.

The diameter of the gradually increasing portion 25 in the vicinity of the opening of the hollow portion represented by "D₃" in Fig. 2(a) is not particularly limited, but is preferably in the range of 2 to 8 mm, more preferably in the range of 4 to 6 mm. By setting the diameter of the gradually increasing portion 25 in the vicinity of each opening of the hollow portion to a value within the above range, the above-described effects becomes more conspicuous.

The fixing member 3 to be used for fixing the interspinous spacer 1 between adjacent spinous processes is not particularly limited, but, for example, a wire-shaped fixing member can be used. Specific examples of such a wire-shaped fixing member 3 include cables made of high molecular polyethylene, sutures made of polyester, and wires made of titanium or stainless steel.

The entire length of the block body 2, having the above-described structure, represented by "L₁" in Fig. 2(a) is preferably in the range of 10 to 40 mm, more preferably in the range of 15 to 25 mm. If the "L₁" is less than the above lower limit value, there is a case where the spacer 1 is not securely held between adjacent spinous processes. On the other hand, if the "L₁" exceeds the above upper limit value, there is a case where it is difficult to insert the spacer 1 between adjacent spinous processes.

The diameter of the block body 2 in the vicinity of each end thereof represented by "L₂" in Fig. 2(a) is preferably in the range of 5 to 20 mm, more preferably in the range of 8 to 15 mm. If the "L₂" is less than the above lower limit value, there is a possibility that the spacer 1 cannot have adequate strength or the spacer 1 is moved off a position desired when strongly shocked. In addition, there is also a possibility that an adequate interspinous space cannot be secured so that a desired therapeutic effect is not obtained. On the other hand, if the "L₂" exceeds the above upper limit value, there is a case where a space between adjacent spinous processes becomes too large depending on the depth of the recess 21.

The block body 2 has rounded-corners (that is, the corners of the block body 2 are chamfered). This makes it possible to more reliably prevent surrounding tissue from being damaged when, for example, the block body 2 is inserted into an interspinous space.

The block body 2 is preferably formed using a biocompatible material. Examples of a biocompatible material include metallic materials less harmful to a living body, such as titanium, titanium alloys, stainless steel, Co-Cr based alloys, and Ni-Ti based alloys, ceramic materials, and composite materials of two or more of them.

Among these materials, titanium and titanium alloys have high strength, and are therefore advantageous in that abrasion of the block body 2 is suppressed even when stress is repeatedly applied to the block body 2. In addition, the use of titanium or titanium alloys is advantageous in that an X-ray image or the like taken after a surgical operation is not distorted.

In a case where a ceramic material is used as a main material, the block body 2 can have especially excellent biocompatibility. In addition, since a ceramic material has excellent workability, it is easy to adjust the shape and size of the block body 2 by cutting work with a lathe, a drill, or the like. It is also possible to fine-adjust the size of the block body 2 in an operation site according to the size of each of the spinous processes 101 and 102, the degree of curvature of the spine, and the like, that is, according to the disease state of a patient.

Although various ceramic materials can be used for the block body 2, alumina, zirconia, bioceramics such as calcium phosphate-based compounds are preferable. Among them, calcium phosphate-based compounds are particularly preferable as a constituent material of the block body 2 because they have excellent biocompatibility.

Examples of a calcium phosphate-based compound include apatites such as hydroxyapatite, fluorapatite, and carbonate apatite, dicalcium phosphate, tricalcium phosphate, tetracalcium phosphate, and octacalcium phosphate. These calcium phosphate-based compounds can be used singly or in combination of two or more of them. Among these calcium phosphate-based compounds, one having a Ca/P ratio of 1.0 to 2.0 is preferably used.

Among such calcium phosphate-based compounds, hydroxyapatite is more preferably used. Hydroxyapatite has the same composition, structure, and physical properties as the major inorganic constituent of bone, and is therefore very highly biocompatible.

Further, hydroxyapatite particles to be used as a raw material for manufacturing the block body 2 are preferably calcined at 500 to 1000°C. By calcining hydroxyapatite particles at 500 to 1000°C, it is possible to suppress activity to a certain degree, thereby suppressing nonuniform sintering caused by, for example, rapid progress of sintering. As a result, a sintered body having uniform strength can be obtained.

The porosity of the thus obtained block body 2 is preferably 50% or less, more preferably in the range of about 0 to 20%. By setting the porosity of the block body 2 to a value within the above range, it is possible to allow the block body 2 to have more appropriate strength.

It is to be noted that the block body 2 can be composed of, for example, composite materials of the above-described ceramic materials and metallic materials less harmful to a living body, such as titanium, titanium alloys, stainless steel, Co-Cr based alloys, and Ni-Ti based alloys.

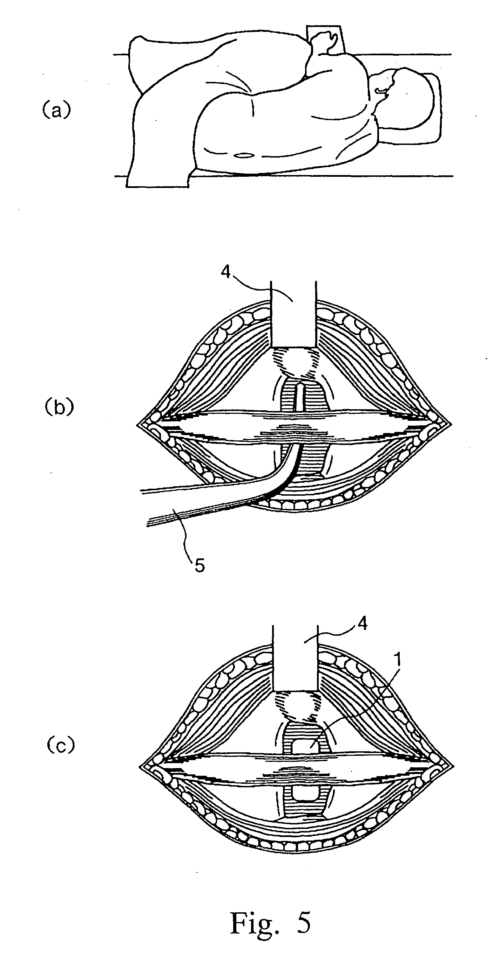

Hereinbelow, an example of a surgical technique for inserting the interspinous spacer of the present invention into an interspinous space will be described.
1 First, as shown in Fig. 5(a), a patient is placed in the flexed right lateral decubitus position, and is locally anesthetized.
2 A skin incision of about 5 cm is made.
3 Muscles are separated from both sides of spinous processes, and are then elevated with a retractor 4.
4 The tip of a curved probe 5 is inserted anterior to a supraspinal ligament (see Fig. 5(b)).
5 The position of a space between the spinous processes is checked with an X-ray image, and the space is widened.
6 The interspinous spacer 1 is inserted into a space between the spinous processes from the right side of the patient (see Fig. 5(c)).
7 The fixing member 3 is passed through the hollow portion 22 of the interspinous spacer 1 to fix the spacer 1 between the spinous processes (see Fig. 3).
8 The operative wound is closed.

As described above, the use of the interspinous spacer according to the present invention allows a less invasive operation to be easily carried out without extensive resection of bone or soft tissue.

The interspinous spacer according to the present invention has been described above with reference to the embodiment shown in the drawings, but the present invention is not limited thereto.

For example, in the above-described embodiment, the block body 2 having a substantially cylindrical shape has been described, but the block body 2 may have any shape such as a substantially spherical shape or a polygonal prismatic shape (e.g., a substantially trigonal prismatic shape, a substantially tetragonal prismatic shape) as long as it includes the recess 22 as described above.

Further, in the above-described embodiment, the recess 21 provided around the entire circumference of the block body 2 has been described, but the recess 21 is not particularly limited as long as it is provided in at least a portion which is brought into contact with the upper and lower spinous processes.

Furthermore, in the above-described embodiment, the interspinous spacer 1 having the hollow portion 22 has been described, but the interspinous spacer 1 does not always have to have the hollow portion 22.

Moreover, in the above-described embodiment, the block body 2 composed of a biocompatible material has been described, but the block body 2 is not limited thereto. For example, at least the surface and its vicinity of the block body 2 may be composed of the material as described above.

Moreover, in the above-described embodiment, the interspinous spacer 1 fixed to one spinous process has been described, but the interspinous spacer 1 may be fixed to both of the upper and lower spinous processes.

### Effects of the Invention

As described above, according to the present invention, it is possible to provide an interspinous spacer to be reliably held between adjacent spinous processes to maintain an appropriate space between the adjacent spinous processes.

Particularly, the interspinous spacer according to the present invention allows a less invasive operation to be easily carried out without extensive resection of bone or soft tissue.

It is to be noted that this application is based on Japanese Patent Application No. 2003-139471, the entire disclosure of which is incorporated herein by reference.

## Claims

1. An interspinous spacer adapted to be used by being inserted between adjacent spinous processes, the interspinous spacer comprising:
a block body having a recess for receiving a part of each of the spinous processes in a state that the block body is inserted between the adjacent spinous processes.

2. The interspinous spacer as claimed in claim 1, wherein the recess is provided around the entire circumference of the block body.

3. The interspinous spacer as claimed in claim 1 or 2, wherein the recess has an arc-shaped bottom.

4. The interspinous spacer as claimed in any one of claims 1 to 3, wherein the depth of the recess is in the range of 0.5 to 10 mm.

5. The interspinous spacer as claimed in any one of claims 1 to 4, wherein the width of the recess is in the range of 1 to 15 mm.

6. The interspinous spacer as claimed in any one of claims 1 to 5, wherein the block body is formed into a substantially cylindrical shape.

7. The interspinous spacer as claimed in any one of claims 1 to 6, wherein the block body is formed with a hollow portion used for passing a fixing member when the interspinous spacer is fixed between the adjacent spinous processes.

8. The interspinous spacer as claimed in any one of claims 1 to 7, wherein the block body has rounded-corners.

9. The interspinous spacer as claimed in any one of claims 1 to 8, wherein the porosity of the block body is 50% or less.

10. The interspinous spacer as claimed in any one of claims 1 to 9, wherein the block body is formed of a material having biocompatibility.

11. The interspinous spacer as claimed in any one of claims 1 to 10, wherein the block body is mainly formed of a ceramic material.

12. The interspinous spacer as claimed claim 11, wherein the ceramic material is a calcium phosphate-based compound.

13. The interspinous spacer as claimed claim 12, wherein the calcium phosphate-based compound has a Ca/P ratio of 1. 0 to 2.0.

14. The interspinous spacer as claimed claim 13, wherein the calcium phosphate-based compound is hydroxyapatite.
